# EUROPEAN PATENT SPECIFICATION

(11) **EP 3 408 406 B1**
(45) Date of publication and mention of the grant of the patent: **15.06.2022**
(21) Application number: 17701499.0
(22) Date of filing: 26.01.2017
(51) Int. Cl.: C12Q 1/6806, C12Q 1/6855, C12Q 1/6869

(54) **A NOVEL Y-SHAPED ADAPTOR FOR NUCLEIC ACID SEQUENCING AND METHOD OF USE**
NEUARTIGER Y-FÖRMIGER ADAPTER ZUR NUKLEINSÄURESEQUENZIERUNG UND VERFAHREN ZUR VERWENDUNG
NOUVEL ADAPTATEUR DU TYP Y DE SÉQUENÇAGE D'ACIDES NUCLÉIQUES ET PROCÉDÉ D'UTILISATION

(30) Priority: 29.01.2016 US 201662288903 P
(43) Date of publication of application: 05.12.2018
(73) Proprietor: F. Hoffmann-La Roche AG, 4070 Basel (CH); Roche Diagnostics GmbH, 68305 Mannheim (DE)
(72) Inventor: KLASS, Daniel, Lake Bluff, Illinois 60044 (US)
(74) Representative: Burger, Alexander
(86) International application number: PCT/EP2017/051588
(87) International publication number: WO 2017/129647

(56) References cited:
- WO-A1-2013/181170
- WO-A1-2015/100427
- WO-A1-2016/040901
- WO-A1-2016/176091
- WO-A2-2009/133466
- US-A1- 2013 034 546

## Description

### Field of the invention

The invention related to nucleic acid analysis, more specifically to adaptors that aid in nucleic acid sequencing.

### Background of the invention

The latest methods of nucleic acid sequencing such as Massive Parallel Sequencing (MPS) also known as Next Generation Sequencing (NGS) involve analysis of individual molecules in a sample. Analysis of each molecule in the sample requires universal primers. Furthermore, part of single molecule analysis is molecular tagging or barcoding whereby each molecule carries information about its origin and its identity. Universal primer binding sites and barcodes can be added to target molecules in a sample by adding an adaptor. Adaptors can be added by extending a primer containing the adaptor sequence or by ligating the adaptor.

A molecular tag or barcode is a short sequence containing unique identifying information. The tag may be unique to a particular sample (shared by all molecules derived from the sample) or used to identify an individual molecule (shared only by progeny of that molecule). The sample ID tags (SID) and unique molecular ID tags (UID) are known in the art. The sample ID allows one to pool samples in a sequencing run while the molecular IDs enable tracking progeny of each molecule in the original sample.

WO2013/181170 discloses Y shaped adaptors which contain randomised indexing adapters in each single stranded portion of the Y-shaped adaptor. The indexes are designed based on the Hamming tag.

WO2015/100427 discloses Y-shaped adaptors wherein the single stranded portions may comprise a strand-identification barcode. The adaptors can be used in determining errors during sequencing.

The present invention employs an economical adaptor that allows for reduced-error nucleic acid sequencing with a minimum expenditure of resources and maximum sensitivity.

### Summary of the invention

The invention is a pool of adaptors, each adaptor comprising a double-stranded portion at one end and a single stranded portion comprising two non-hybridizable strands at the opposite end, and further comprising at least one primer-binding site and at least one barcode in each single-stranded portion, wherein the barcodes on each adaptor in the pool are in a known relationship. The barcodes on one strand of the same adaptor may be at least one edit distance apart. The relationship between the barcodes on the same adaptor is reverse complementarity.

In another embodiment, the invention is an article of manufacture comprising the pool of adaptors described above. The pool may be contained in a single vial.

In yet another embodiment, the invention is a method of sequencing nucleic acids comprising: ligating to each nucleic acid in a sample an adaptor comprising a double-stranded portion at one end and a single stranded portion comprising two non-hybridizable strands at the opposite end, and further comprising at least one primer-binding site and a first barcode on the first strand and a second barcode on the second strand of the single-stranded portion, wherein the first and second barcodes on each adaptor in the pool are in a known relationship, wherin the known relationship is reverse complementarity, determining the sequence of at least a portion of the nucleic acid strands and of the first and second barcodes, comparing the sequence of the nucleic acid strand containing the first barcode and the sequence of the nucleic acid strand containing the second barcode to identify not perfectly complementary sequences, determining that the not perfectly complementary sequences contain at least one experimental error. The method may further comprise amplifying the ligated nucleic acid prior to sequence determination to obtain separate double stranded sequences containing the first and the second barcode. The sequences determined to contain at least one experimental error may be omitted from the sequencing results. The method may further comprise grouping sequences containing the same barcode and the same genomic coordinates of the nucleic acid, comparing sequences within the group to identify non-identical sequences and determining that the non-identical sequences contain at least one experimental error. The sample used in the method may contain cell-free DNA.

Outside the scope of the invention is a method of making a pool of adaptors for nucleic acid sequencing comprising annealing in a pairwise manner single strands of nucleic acid to form adaptors comprising a double-stranded portion at one end and a single stranded portion comprising two non-hybridizable strands at the opposite end, and further comprising at least one primer-binding site and at least one barcode in each single-stranded portion, wherein prior to annealing the single strands of nucleic acids are combined in a way that establishes a known relationship between the barcodes in the pool of adaptors.

### Brief description of the drawings

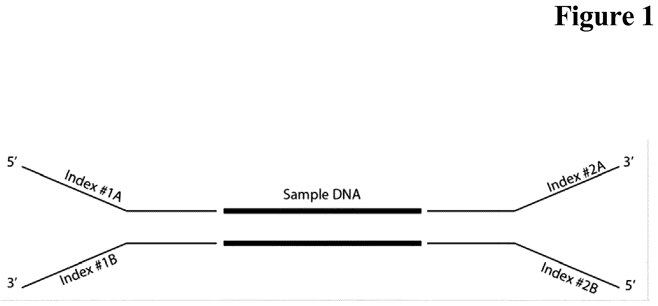

FIG. 1: A diagram of the adaptors ligated to both ends of a sample nucleic acid.

### Detailed description of the invention

### Definitions

The term "adaptor" refers to a polynucleotide that can be attached to one or both termini of a nucleic acid molecule. An adaptor may comprise only a double-stranded region or also a single-stranded region. The double-stranded region is formed by hybridizable portions of two nucleic acid strands while the single-stranded region is formed by non-hybridizable portions of the same two nucleic acid strands. The non-hybridizable portion may be open (Y-shaped adaptor) or covalently closed by linking the free 5'- and 3'-ends (dumbbell-shaped adaptor). In the case of a Y-shaped adaptor, the single-stranded portion of the adaptor is sometimes referred to as a "fork," while the double stranded portion is sometimes referred to as a "stem."

The terms "barcode " and "index" are used interchangeably to refer to a sequence of nucleotides within a polynucleotide that is used to identify a nucleic acid molecule. For example, a barcode can be used to identify a sample from which a nucleic acid molecule is derived when several samples are combined (as is common in some massively parallel sequencing techniques). A barcode can also be used to identify a unique nucleic acid molecule and progeny thereof resulting from amplification. A barcode can be synthesized at the time a nucleic acid (*e.g*., a primer or an adaptor) is synthesized. A barcode can comprise pre-defined or random sequences or combinations thereof. The term "pre-defined" means that sequence of a barcode is known at the time a nucleic acid with the barcode is synthesized. The term "random " or "degenerate sequence" means that a random mixture of nucleotides is used when the barcode within the nucleic acid is synthesized. A non-random, i.e., biased mixture of bases can be used during oligonucleotide sequencing resulting in a barcode that preferentially contains certain bases. A barcode can sometimes comprise an endogenous sequence present in the unaltered genome. An endogenous barcode can be formed by a junction of the randomly fragmented nucleic acid and an adaptor. A combination synthetic-endogenous barcode can be formed by the combination of the genomic coordinates of the start and end position of the randomly fragmented nucleic acid and a synthetic barcode in the adaptor.

The term "single-stranded barcode," e.g., within an adaptor, means a barcode not hybridized to its complementary sequence. A "double-stranded barcode" means a barcode hybridized to its complementary sequence. For example, a single-stranded barcode can be situated in the single-stranded portion of an adaptor, and a double-stranded barcode can be situated in the double-stranded portion of an adaptor.

The term "hybridizable " refers to two polynucleotide strands that can form a duplex. The duplex can form when the strands are perfectly or at least partially complementary. Complementarity may be defined by Watson-Crick hydrogen bonding. Additional interactions (e.g., Hoogsteen pairing and hydrophobic interactions) can support hybridization in the absence of perfect Watson-Crick complementarity.

The term "non-hybridizable" refers to two polynucleotide strands that cannot form a duplex under experimental conditions. The duplex is unable to form when the strands do not share even partial complementarity and no additional interactions (e.g., Hoogsteen pairing and hydrophobic interactions) suffice to support specific hybridization.

The term "edit distance" between two nucleic acid sequences, especially between two barcodes, refers to the number of changes required to change one sequence into another, where a change is the addition, subtraction, or substitution of a base.

The term "paired" in reference to barcodes means having a known relationship between two barcode sequences on the two oligos of an adaptor molecule. The term includes complementarity (base pairing), reverse complementarity, as well as any other artificial relationship, e.g., a reference table, indicating which two barcoded adaptor strands have been intentionally paired during the hybridization step.

The term "amplification" refers to any method for increasing the number of copies of a nucleic acid sequence. For example, the amplification can be performed with the use of a polymerase, e.g., in one or more polymerase chain reactions (PCR) or another exponential or linear method of amplification. The term "amplicons " means nucleic acid products of an amplification reaction.

The terms "universal primer" and "universal primer site" refer to a primer and a primer-binding sequence not present in any target sequence but added to all target sequences (e.g., by being a part of a target-specific primer or by being a part of an adaptor). After the universal primer site has been added, the universal primer can be used for amplification or sequencing of all target sequences in a sample.

The term "deduping" refers to a method of grouping nucleic acid sequences into groups consisting of progeny of a single molecule originally present in the sample. Deduping further comprises analysis of the sequences of the progeny molecules to indirectly determine the sequence of the original molecule with a reduced rate of errors.

The term "error" in the context of nucleic acid sequencing refers to an incorrect base readout. The term encompasses any error revealed during the sequencing step, not only the error of the sequencing step itself. The error includes errors of DNA polymerase during primer extension or target amplification, errors of the sequencing polymerase and errors of the sequencing instrument, e.g., detector. Where an artificial sequence is being read (e.g., adaptor sequence), errors also include errors of *in vitro* DNA synthesis (oligo synthesis). Errors include base substitution (wrong base),lack of incorporation (deleted base), or addition of a base (inserted base). The term "error rate" refers to the number of errors per correct base read. The term "reduced error rate" from an error-prevention measure refers to the error rate with the measure compared to the error rate without the measure.

The term "cell-free DNA (cfDNA)" refers to DNA in a sample that when collected, was not contained within a cell. The term does not refer to DNA that is rendered cell-free by *in vitro* disruption of cells or tissues. cfDNAs can comprise both normal cell and cancer cell-derived DNA. cfDNA is commonly obtained from blood or plasma ("circulation"). cfDNAs may be released into the circulation through secretion or cell death processes, e.g., cellular necrosis or apoptosis. Some cfDNA is ctDNA (see below).

The term "circulating tumor DNA (ctDNA)" or "circulating cancer DNA" refers to the fraction of cell-free DNA (cfDNA) that originates from a tumor.

The term "sample" refers to any biological sample that is isolated from a subject. For example, a sample can include body tissues or fluids. The sample may also be a tumor sample. Samples can be obtained directly from a subject, from previously excised or drawn sample or from the environment (e.g., forensic samples).

The term "blood sample " refers to whole blood or any fraction thereof, including blood cells, serum and plasma.

The invention includes adaptors for single-molecule sequencing of nucleic acids. Adaptors conjugated to a nucleic acid molecule are shown in Figure 1. The current nucleic acid sequencing methods, referred to as Next Generation Sequencing (NGS) or Massively Parallel Sequencing (MPS) involve capturing, optionally amplifying and sequencing each individual molecule in a sample. Optional amplification can be before capture, after capture, or both. NGS further involves universal sequencing primers and optionally, universal pre-amplification primers. To create binding sites for universal primers, each target nucleic acid molecule is conjugated to an adaptor. Adaptors are typically conjugated to both sides of target nucleic acid molecules and contain binding sites for universal primers and other sequences necessary for sequencing. Adaptors may contain barcodes that uniquely identify a sample from which target molecules originated (sample ID or SID). Adaptors may contain barcodes that uniquely identify each target molecules (unique molecular ID or UID). SID and UID may exist separately or be combined into a single barcode.

A convenient way to attach adaptors to a double-stranded target nucleic acid is via ligation. For a ligation reaction to occur, the target nucleic acid and the adaptor must have compatible ends. In some embodiments, the target nucleic acid is end-repaired to contain blunt ends and the adaptor has a double stranded blunt end. In other embodiments, the target nucleic acid is end-repaired and both the target nucleic acid and the adaptor are engineered to have a one-base extension. For example, and extension creating a T-A pair enables efficient ligation between the adaptor molecule and the target nucleic acid. DNA overhangs resulting from a restriction digest could also be used to improve ligation efficiency.

Especially advantageous are Y-shaped adaptors described e.g., in U.S. Patent No. 6395887. These adaptors comprise a double-stranded portion at one end and a single stranded portion comprising two non-hybridizable strands at the opposite end. Only the double-stranded portion is capable of ligation to the target nucleic acid ensuring correct orientation of the ligated products.

In one embodiment, the invention is a novel adaptor for analysis of nucleic acids. (Figure 1). The adaptor comprises a double-stranded portion at one end and a single stranded portion comprising two non-hybridizable strands at the opposite end. The precise length of each portion is not essential as long as the adaptor possesses the following properties: 1) has sufficient length to accommodate all the elements described below; 2) has a suitable melting temperature; and 3) does not form any secondary structure in the single-stranded portion that may impede the adaptor's performance. One skilled in the art can design an oligonucleotide with desired melting temperature to accommodate a particular assay needs. Likewise, at least some secondary structure formation can be avoided or mitigated by one skilled in the art using state of the art oligonucleotide design tools. In some embodiments, it is desired that the length of the single-stranded portion not exceed 20 nucleotides and the length of the double stranded be sufficient to remain hybridized at room temperature and allow binding of DNA ligase.

The adaptor comprises binding sites for one or more primers. The primers may be sequencing primers, amplification primers or both. In some embodiments, the same primer may be a sequencing primer and an amplification primer. The adaptors may also comprise sequences specific to a particular sequencing technology, for example, sequences that hybridize to the solid support in the sequencing instrument (*e.g*., cluster generation sequences in Illumina instruments).

The adaptor may contain, naturally occurring bases (*e.g*., Adenosine (A), Thymidine (T), Guanosine (G), Cytosine (C), and Uracil (U)), other natural bases such as Inosine (I) and methyl-Cytosine (mC), modified versions of the natural bases as well as non-naturally occurring bases *e.g*., aminoallyl-uridine, iso-cytosines, isoguanine, and 2-aminopurine.

The adaptors used in the present invention further comprise barcodes. The barcode can contain natural or non-natural nucleotides described above. The barcode may have a pre-defined sequence, a random sequence, or a non-random biased sequence that preferentially contains certain bases. In some embodiments, a biased sequence is used to avoid error-prone bases. In other embodiments, a biased sequence is used to modulate the melting temperature of the barcode-containing nucleic acid. As shown in Figure 1, each adaptor comprises two barcodes or indices, one on each of the single strands of the single-stranded portion. The ligated product comprising a target DNA fragment and two adaptors comprises four barcodes. The barcodes in each adaptor (e.g., Index 1A and 1B) have sequences in a 1:1 relationship. The relationship is reverse complementarity; whereby identifying one barcode sequence (e.g., Index 1A) unambiguously determines the second barcode sequence (Index 1B).

In some embodiments, the invention is a pool of adaptors described in Figure 1. In the pool each adaptor comprises a double-stranded portion at one end and a single stranded portion comprising two non-hybridizable strands at the opposite end. The adaptors in the pool further comprise binding sites for one or more primers, e.g., sequencing primers, amplification primers or both. The adaptors in the pool further comprise barcodes. Specifically, each adaptor comprises two barcodes, one on each of the single strands of the single-stranded portion. The barcodes in adaptors are in a 1:1 relationship whereby identifying one barcode sequence unambiguously determines the second barcode sequence. The sequences are reverse complementary, .

The adaptors within the pool have barcodes at least 1 or at least 3 edit distance apart. One of skilled in the art would be able to determine what edit distance is optimal for a particular experiment. Generally, greater edit distance means that fewer barcodes can be used in one pool. However, if an assay or a manufacturing process has a high error rate, greater edit distance will be required. For example, oligonucleotide manufacturing process used to make adaptors may have a high error rate. Similarly, a nucleic acid polymerase used in DNA amplification or primer extension in the sequencing by synthesis workflow can have a high error rate. These error rates would require increasing edit distance among the barcodes in adaptors of the pool. Conversely, improving the accuracy of each of the methods mentioned above will allow decreasing edit distance among the barcodes in adaptors of the pool.

In some embodiments, the invention is a pool of N distinct adaptors each consisting of two annealed oligonucleotides (2N oligonucleotides in the pool.) Depending on the length of the barcodes in the adaptors, each sample will require a pool consisting of A adaptors. Therefore the pool of N can be used in N/A=S samples. In some embodiments, an article of manufacture may comprise a single vial containing the entire pool of adaptors. Alternatively, an article of manufacture can comprise a kit where one or more adaptors of the pool are present in separate vials.

Outside the scope of the invention is a method of making adaptors for nucleic acid analysis. The method comprises combining and annealing in a pairwise manner two single strands of nucleic acid to form adaptors wherein each adaptor comprises a double-stranded portion at one end and a single stranded portion comprising two non-hybridizable strands at the opposite end. The single strands forming the adaptors comprise binding sites for one or more primers, e.g., sequencing primers, amplification primers or both. The single strands forming the adaptors further comprise barcodes. Specifically each strand comprises a barcode in the non-complementary region so that each adaptor comprises at least two barcodes, At least one on each of the single strands of the single-stranded portion. The single strands are combined and annealed so that barcodes in adaptors are in a 1:1 relationship. The sequences are reverse complementary, . Thus, adaptors are used in a method that involves creating a reference whereby identifying one sequence (e.g., Index 1A in Fig. 1) unambiguously determines the second sequence (Index 1B in Fig. 1).

In some embodiments, the invention is a method of sequencing nucleic acids in a sample using adaptors with single-stranded barcodes. The method comprises attaching to nucleic acids in the sample a pool of adaptors to form a pool of adaptor-target molecules. The attaching may be via ligation with a DNA ligase, *e.g*., a T4 DNA ligase, *E. coli* DNA ligase, mammalian ligase, or any combination thereof. The mammalian ligase may be DNA ligase I, DNA ligase III, or DNA ligase IV. The ligase may also be a thermostable ligase. In some embodiments, to increase the efficiency of ligation, the sample nucleic acid may be subjected to end repair (e.g., with a DNA polymerase) and A-tailing, also with a DNA polymerase or terminal transferase.

Each adaptor comprises a double-stranded portion at one end and a single stranded portion comprising two non-hybridizable strands at the opposite end. The adaptor comprises a first barcode in one strand of the single stranded portion and a second barcode in the other strand of the single stranded portion, and wherein the first and second barcodes in each adaptor are in a known relationship , whereby the known relationship is reverse complementarity, such that each first barcode can be unambiguously associated with each second barcode. In each sample, multiple adaptors with multiple pairs of barcodes are present but there are fewer adaptors then target nucleic acid molecules in each sample. Yet the number of adaptors with unique pairs of barcodes is sufficient to identify all, nearly all, or a desired percentage of the original nucleic acid molecules in the sample. The identification utilizes both the unique barcode and the genomic coordinates (breakpoints) for each target nucleic acid molecule as described below. The adaptor further comprises binding sites for one or more primers. In some embodiments, the method further comprises a step of amplifying both strands of the adaptor-target molecules prior to determining their sequence. The method further comprises a step of determining the sequence of the adaptor-target molecules. In this step, at least a portion of the sequence of the target nucleic acid is determined and the sequence of barcodes in the adaptors is determined. The method further comprises a step of error correction wherein the adaptor-target sequence containing each first barcode is paired with the adaptor-target sequence containing the corresponding second barcode in the known relationship with the first barcode. As shown in Figure 1, the target sequence attached to the adaptor with barcode 1A is paired with the target sequence attached to the adaptor with barcode 1B. The first molecules with barcode 1A represent the first strand of the original molecule and the second molecules with barcode 1B represent the second strand of the original molecule. Pairing barcodes 1A and 1B allows matching of the original strands for error correction. If the target sequence of the first and the second molecules is not identical, e.g., a base substitution is present in only the first but not the second molecules, the change is deemed to be an experimental error. Molecules containing experimental errors are omitted from the results. In some embodiments, the molecules containing experimental error found in the raw data file are not included in the results file. Same-origin sequences are also identified by virtue of having the same adaptor barcodes and the same genomic coordinates of the target nucleic acid. If the target sequence of the same-origin molecules is not identical, e.g., a base substitution is present in only a fraction of the same-origin molecules, the change is deemed to be an experimental error.

In some embodiments, the sample comprises cell-free nucleic acids, such as cell-free plasma nucleic acids. Such DNA may be fragmented, e.g., may be on average about 170 nucleotides in length, which may coincide with the length of DNA wrapped around a single nucleosome. In embodiments where the sample nucleic acid is not naturally fragmented, the nucleic acid can be fragmented *in vitro* using *e.g*., sonication or restriction digestion.

## Claims

1. A pool of adaptors, each adaptor comprising a double-stranded portion at one end and a single stranded portion comprising two non-hybridizable strands at the opposite end, and further comprising at least one primer-binding site and at least one barcode in each single-stranded portion, wherein the barcodes on each adaptor in the pool are in a known relationship,
wherein the relationship between the barcodes is reverse complementarity,

2. An article of manufacture comprising the pool of adaptors of claim 1.

3. The article of claim 2, wherein the pool is contained in a single vial.

4. A method of sequencing nucleic acids comprising:
a) ligating to each nucleic acid in a sample an adaptor from a pool of adaptors according to claim 1 comprising a double-stranded portion at one end and a single stranded portion comprising two non-hybridizable strands at the opposite end, and further comprising at least one primer-binding site and a first barcode on the first strand and a second barcode on the second strand of the single-stranded portion, wherein the first and second barcodes on each adaptor in the pool are in a known relationship, wherein the relationship between the barcodes is reverse complementarity,
b) determining the sequence of at least a portion of the nucleic acid strands and of the first and second barcodes,
c) comparing the sequence of the nucleic acid strand containing the first barcode and the sequence of the nucleic acid strand containing the second barcode to identify not perfectly complementary sequences,
d) determining that the not perfectly complementary sequences contain at least one experimental error.

5. The method of claim 4, further comprising amplifying the ligated nucleic acid prior to sequence determination to obtain separate double stranded sequences containing the first and the second barcode.

6. The method of claim 4, wherein the sequences determined to contain at least one experimental error are omitted from the sequencing results.

7. The method of claim 4, further comprising grouping sequences containing the same barcode and the same genomic coordinates of the nucleic acid, comparing sequences within the group to identify non-identical sequences and determining that the non-identical sequences contain at least one experimental error.

8. The method of claim 4, wherein the sample contains cell-free DNA.

## Patentansprüche

1. Pool von Adaptoren, wobei jeder Adaptor einen doppelsträngigen Abschnitt an einem Ende und einen einzelsträngigen Abschnitt, der zwei nicht hybridisierbare Stränge umfasst, am entgegengesetzten Ende umfasst und ferner mindestens eine Primer-Bindungsstelle und mindestens einen Barcode in jedem einzelsträngigen Abschnitt umfasst, wobei die Barcodes an jedem Adaptor in dem Pool in einer bekannten Beziehung stehen,
wobei die Beziehung zwischen den Barcodes umgekehrte Komplementarität ist.

2. Erzeugnis, umfassend den Pool von Adaptoren nach Anspruch 1.

3. Erzeugnis nach Anspruch 2, wobei der Pool in einem einzelnen Fläschchen enthalten ist.

4. Verfahren zum Sequenzieren von Nukleinsäuren, umfassend:
a) Ligieren eines Adaptors aus einem Pool von Adaptoren nach Anspruch 1, der einen doppelsträngigen Abschnitt an einem Ende und einen einzelsträngigen Abschnitt, der zwei nicht hybridisierbare Stränge umfasst, am entgegengesetzten Ende umfasst und ferner mindestens eine Primer-Bindungsstelle und einen ersten Barcode an dem ersten Strang und einen zweiten Barcode an dem zweiten Strang des einzelsträngigen Abschnitts umfasst, an jede Nukleinsäure in einer Probe, wobei der erste und der zweite Barcode an jedem Adaptor in dem Pool in einer bekannten Beziehung stehen, wobei die Beziehung zwischen den Barcodes umgekehrte Komplementarität ist,
b) Bestimmen der Sequenz von mindestens einem Abschnitt der Nukleinsäurestränge und des ersten und zweiten Barcodes,
c) Vergleichen der Sequenz des Nukleinsäurestranges, der den ersten Barcode enthält, und der Sequenz des Nukleinsäurestranges, der den zweiten Barcode enthält, um nicht perfekt komplementäre Sequenzen zu identifizieren,
d) Bestimmen, dass die nicht perfekt komplementären Sequenzen mindestens einen experimentellen Fehler enthalten.

5. Verfahren nach Anspruch 4, ferner umfassend das Amplifizieren der ligierten Nukleinsäure vor der Sequenzbestimmung, um separate doppelsträngige Sequenzen zu erhalten, die den ersten und den zweiten Barcode enthalten.

6. Verfahren nach Anspruch 4, wobei die Sequenzen, die nachweislich mindestens einen experimentellen Fehler enthalten, aus den Sequenzierungsergebnissen weggelassen werden.

7. Verfahren nach Anspruch 4, ferner umfassend das Gruppieren von Sequenzen, die denselben Barcode und dieselben genomischen Koordinaten der Nukleinsäure enthalten, das Vergleichen von Sequenzen innerhalb der Gruppe, um nicht identische Sequenzen zu identifizieren, und das Bestimmen, dass die nicht identischen Sequenzen mindestens einen experimentellen Fehler enthalten.

8. Verfahren nach Anspruch 4, wobei die Probe zellfreie DNA enthält.

## Revendications

1. Groupe d'adaptateurs, chaque adaptateur comprenant une partie double brin à une extrémité et une partie simple brin comprenant deux brins non hybridables à l'extrémité opposée, et comprenant en outre au moins un site de liaison à l'amorce et au moins un code-barres dans chaque partie simple brin, dans lequel les codes-barres sur chaque adaptateur dans le groupe sont dans une relation connue,
dans lequel la relation entre les codes-barres est une complémentarité inverse.

2. Article manufacturé comprenant le groupe d'adaptateurs selon la revendication 1.

3. Article selon la revendication 2, dans lequel le groupe est contenu dans un flacon unique.

4. Procédé de séquençage d'acides nucléiques comprenant :
a) la ligature à chaque acide nucléique dans un échantillon d'un adaptateur d'un groupe d'adaptateurs selon la revendication 1 comprenant une partie double brin à une extrémité et une partie simple brin comprenant deux brins non hybridables à l'extrémité opposée, et comprenant en outre au moins un site de liaison à l'amorce et un premier code-barres sur le premier brin et un second code-barres sur le second brin de la partie simple brin, les premier et second codes-barres sur chaque adaptateur dans le groupe étant dans une relation connue, la relation entre les codes-barres étant une complémentarité inverse,
b) la détermination de la séquence d'au moins une partie des brins d'acides nucléiques et des premier et second codes-barres,
c) la comparaison de la séquence du brin d'acide nucléique contenant le premier code-barres et de la séquence du brin d'acide nucléique contenant le second code-barres pour identifier des séquences non parfaitement complémentaires,
d) la détermination que les séquences non parfaitement complémentaires contiennent au moins une erreur expérimentale.

5. Procédé selon la revendication 4, comprenant en outre l'amplification de l'acide nucléique ligaturé avant la détermination de séquence pour obtenir des séquences double brin séparées contenant le premier et le second code-barres.

6. Procédé selon la revendication 4, dans lequel les séquences déterminées pour contenir au moins une erreur expérimentale sont omises des résultats de séquençage.

7. Procédé selon la revendication 4, comprenant en outre le regroupement de séquences contenant le même code-barres et les mêmes coordonnées génomiques de l'acide nucléique, la comparaison des séquences au sein du groupe pour identifier les séquences non identiques et la détermination que les séquences non identiques contiennent au moins une erreur expérimentale.

8. Procédé selon la revendication 4, dans lequel l'échantillon contient un ADN acellulaire.
